# EUROPEAN PATENT APPLICATION

(11) **EP 3 951 394 A1**
(43) Date of publication of application: **09.02.2022**
(21) Application number: 20784529.8
(22) Date of filing: 16.03.2020
(51) Int. Cl.: G01N 33/543

(54) **IMMUNOCHROMATOGRAPHY**

(30) Priority: 29.03.2019 JP 2019066094
(71) Applicant: FUJIFILM Corporation, Tokyo 106-8620 (JP)
(72) Inventor: ABURAYA, Yoshihiro, Ashigarakami-gun, Kanagawa 258-8538 (JP); NISHIKAWA, Naoyuki, Ashigarakami-gun, Kanagawa 258-8538 (JP); KATADA, Junichi, Ashigarakami-gun, Kanagawa 258-8538 (JP); UJIHARA, Dai, Ashigarakami-gun, Kanagawa 258-8538 (JP); WADA, Atsuhiko, Ashigarakami-gun, Kanagawa 258-8538 (JP)
(74) Representative: Jeffrey, Philip Michael
(86) International application number: PCT/JP2020/011491
(87) International publication number: WO 2020/203228

(57) **Abstract**

An object of the present invention is to provide immunochromatography exhibiting excellent magnetic detection sensitivity. The immunochromatography of the present invention is immunochromatography including a mixing step of mixing a specimen that is capable of containing a test substance with a modified composite particle that is a composite particle modified with a first binding substance that is capable of binding to the test substance, to obtain a complex of the test substance in the specimen and the modified composite particle; a developing step of developing the complex on an insoluble carrier having a reaction site at which a second binding substance capable of binding to the test substance is immobilized; a capturing step of capturing the complex at the reaction site of the insoluble carrier; and a silver amplification step of silver-amplifying the complex captured in the capturing step, where the composite particle is a composite particle of a magnetic particle and a gold particle carried on a surface of the magnetic particle.

## Description

### BACKGROUND OF THE INVENTION

### 1. Field of the Invention

The present invention relates to immunochromatography.

### 2. Description of the Related Art

Immunochromatography is frequently used these days since the operation is easy and measurement can be carried out in a short time.

For example, in a case where an antigen such as influenza virus is detected by immunochromatography, the following operation is carried out.

First, a label modified with an antibody (labeled antibody) is prepared and mixed with a specimen containing an antigen. The labeled antibody binds to an antigen, whereby a complex is formed. In a case where this complex is developed on an insoluble carrier having a detection line (a test line) to which an antibody that specifically reacts with an antigen is applied, the complex reacts with the antibody on the detection line and is captured, and detection is confirmed visually or in other manners.

Under such circumstances, JP2007-205911A proposes a composite magnetic particle of gold and iron oxide as a label for immunochromatography. JP2007-205911A describes that in a case where the above label is used, magnetic detection is possible.

### SUMMARY OF THE INVENTION

On the other hand, the inventors of the present invention examined immunochromatography using the composite magnetic particle of gold and iron oxide described in JP2007-205911A as a label, and as a result, it was revealed that the magnetic detection sensitivity does not always satisfy the level required these days.

In consideration of the above circumstances, an object of the present invention is to provide immunochromatography exhibiting excellent magnetic detection sensitivity.

As a result of diligent studies on the above problems, the inventors of the present invention have found that in a case where a magnetic particle having gold particles carried on the surface thereof is used as a label and the complex captured on the test line is silver-amplified in immunochromatography, surprisingly the magnetic detection sensitivity is improved, and have reached the present invention.

That is, the inventors of the present invention have found that the object can be achieved by the following configurations.
(1) Immunochromatography comprising:
   a mixing step of mixing a specimen that is capable of containing a test substance with a modified composite particle that is a composite particle modified with a first binding substance that is capable of binding to the test substance, to obtain a complex of the test substance in the specimen and the modified composite particle;
   a developing step of developing the complex on an insoluble carrier having a reaction site at which a second binding substance capable of binding to the test substance is immobilized;
   a capturing step of capturing the complex at the reaction site of the insoluble carrier; and
   a silver amplification step of silver-amplifying the complex captured in the capturing step,
   in which the composite particle is a composite particle of a magnetic particle and a gold particle carried on a surface of the magnetic particle.
(2) The immunochromatography according to (1), in which a material of the magnetic particle is iron oxide.
(3) The immunochromatography according to (1) or (2), further comprising a collection step of collecting the modified composite particle or the complex using magnetism, before the developing step.
(4) The immunochromatography according to any one of (1) to (3), in which the number of carried gold particles of the composite particle is 1 or more and 300 or less.
(5) The immunochromatography according to any one of (1) to (4), in which in the composite particle, an average particle diameter of the gold particles is smaller than an average particle diameter of the magnetic particles.
(6) The immunochromatography according to any one of (1) to (5), in which an average particle diameter of the composite particles is 10 nm to 300 nm.
(7) The immunochromatography according to any one of (1) to (6), in which the silver amplification step is a step of silver-amplifying the complex captured in the capturing step, using a reducing agent solution and a silver amplification solution.
(8) The immunochromatography according to (7), in which an angle between a developing direction of the reducing agent solution in the silver amplification step and a developing direction of the complex in the developing step is 0 degrees to 150 degrees.
(9) The immunochromatography according to (7) or (8), in which an angle between a developing direction of the silver amplification solution in the silver amplification step and a developing direction of the complex in the developing step is 45 degrees to 180 degrees.
(10) The immunochromatography according to any one of (1) to (9), in which both optical detection and magnetic detection are possible.

As described below, according to the present invention, it is possible to provide immunochromatography exhibiting excellent magnetic detection sensitivity.

### BRIEF DESCRIPTION OF THE DRAWINGS

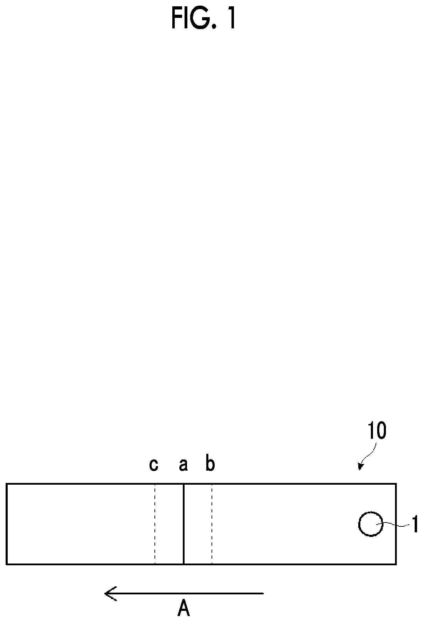

Fig. 1 is a schematic view of a nitrocellulose membrane 10 that is used in Example 1.

### DESCRIPTION OF THE PREFERRED EMBODIMENTS

Immunochromatography according to the embodiment of the present invention will be described below.

In the present specification, the numerical value range indicated by using "to" means a range including the numerical values before and after "to" as the lower limit value and the upper limit value, respectively.

Furthermore, in the present specification, one kind of each component may be used alone, or two or more kinds thereof may be used in combination. In a case where two or more kinds of each component are used in combination, a content of the component indicates a total content unless otherwise specified.

### [Immunochromatography]

The immunochromatography according to the embodiment of the present invention includes the following steps of (1) to (4). The immunochromatography according to the embodiment of the present invention may include steps other than the following steps of (1) to (4).

### (1) Mixing step

A step of mixing a specimen that is capable of containing a test substance with a modified composite particle that is a composite particle modified with a first binding substance that is capable of binding to the test substance, to obtain a complex of the test substance in the specimen and the modified composite particle

### (2) Developing step

A step of developing the complex on an insoluble carrier having a reaction site at which a second binding substance capable of binding to the test substance is immobilized

### (3) Capturing step

A step of capturing the complex at the reaction site of the insoluble carrier

### (4) Silver amplification step

A step of silver-amplifying the complex captured in the capturing step

Here, the composite particle is a composite particle of a magnetic particle and a gold particle carried on a surface of the magnetic particle.

In the following, first, the composite particle that is used in the mixing step will be described, and then each of the steps will be described.

### [Composite particle]

As described above, the composite particle that is used in the mixing step is a composite particle of a magnetic particle and a gold particle carried on the surface of the magnetic particle (hereinafter, also referred to as a "composite particle according to the embodiment of the present invention").

### <Magnetic particle>

The magnetic particle constituting the composite particle of the embodiment of the present invention is not particularly limited as long as it is a particle having magnetism.

Since the composite particle according to the embodiment of the present invention has a magnetic particle, magnetic detection is possible after the capturing step described later. In addition, since the composite particle of the embodiment of the present invention includes a magnetic particle, the composite particle can be easily collected using magnetism.

A material of the magnetic particles is not particularly limited as long as it is a material having magnetism, and specific examples thereof include iron, cobalt, nickel, oxides thereof, ferrite, alloys thereof, and the like. Among them, the material thereof is preferably iron oxide due to the reason that the magnetic detection sensitivity and the optical detection sensitivity in the immunochromatography according to the embodiment of the present invention are more excellent. Hereinafter, "the magnetic detection sensitivity and the optical detection sensitivity in the immunochromatography according to the embodiment of the present invention are more excellent" is also referred to as "the effects and the like of the present invention are more excellent".

The magnetic particle may be a particle obtained by molding only a material having magnetic properties into a particle form. Alternatively, the magnetic particle may be a particle of which the surface has been coated with a polymer (such as polystyrene or silica gel) or the like and which has a material having magnetic properties as a core, or may be a particle of which the surface has been coated using a material having magnetic properties and which has a polymer or the like as a core.

### (Average particle diameter)

The average particle diameter of the magnetic particles is preferably 300 nm or less, more preferably 250 nm or less, still more preferably 200 nm or less, and particularly preferably 100 nm or less, due to the reason that the effects and the like of the present invention are more excellent.

The lower limit of the average particle diameter of the magnetic particles is not particularly limited; however, it is preferably 1 nm or more, more preferably 10 nm or more, still more preferably 20 nm or more, and particularly preferably 40 nm or more, due to the reason that collectability using magnetism is more excellent.

The average particle diameter of the magnetic particles is a value obtained as follows: at least 20 composite particles are observed with a transmission electron microscope (TEM), magnetic particles in the composite particles are specified by energy-dispersive X-ray spectroscopy (EDX), diameters of circles having the same area as a projected area of each of the magnetic particles are individually calculated, and these diameters are arithmetically averaged.

### <Gold particle>

The gold particle constituting the composite particle according to the embodiment of the present invention is not particularly limited.

Due to having gold particles, the composite particle according to the embodiment of the present invention can be optically detected after the capturing step described later. In addition, the gold particle also acts as a catalyst that reduces silver ions in the silver amplification step described later.

### (Average particle diameter)

The average particle diameter of the gold particles is preferably 100 nm or less, more preferably 50 nm or less, still more preferably 30 nm or less, and particularly preferably 15 nm or less, due to the reason that the effects and the like of the present invention are more excellent.

The lower limit of the average particle diameter of the gold particles is not particularly limited; however, it is preferably 1 nm or more, more preferably 2 nm or more, and still more preferably 5 nm or more, due to the reason that the effects and the like of the present invention are more excellent.

The average particle diameter of gold particles is determined by the same method as the method for the magnetic particles described above.

### <Number of carried gold particles>

In the composite particle of the embodiment of the present invention, the average number of gold particles carried on the surface on one magnetic particle (hereinafter, also referred to as "the number of carried gold particles") is not particularly limited; however, it is preferably 500 or less, more preferably 300 or less, still more preferably 100 or less, and particularly preferably 20 or less, due to the reason that the effects and the like of the present invention are more excellent. The lower limit of the number of carried gold particles is not particularly limited; however, it is preferably 1 or more, more preferably 2 or more, and still more preferably 5 or more, due to the reason that the effects and the like of the present invention are more excellent.

The number of carried gold particles is obtained as follows.

First, SEM-EDX analysis is carried out on the composite particle, a signal intensity derived from the magnetic particle and a signal intensity of Au are measured, and an amount ratio between the magnetic particle and the gold particle is determined. Furthermore, the number of carried gold particles is calculated by using the determined amount ratio and the average particle diameters of the magnetic particles and the gold particles which are measured as described above.

### <Average particle diameter of composite particles>

The average particle diameter of the composite particles of the embodiment of the present invention is not particularly limited; however, it is preferably 10 to 600 nm, more preferably 20 to 300 nm, and still more preferably 30 to 100 nm, due to the reason that the effects and the like of the present invention are more excellent.

Here, the average particle diameter of gold particles is preferably smaller than the average particle diameter of magnetic particles due to the reason that the effects and the like of the present invention are more excellent.

The average particle diameter of composite particles is determined by the same method as the method for the magnetic particles described above.

### <Manufacturing method>

The method for manufacturing the composite particle according to the embodiment of the present invention is not particularly limited; however, it is preferably a method in which core particles made of a magnetic iron oxide are dispersed in a solution containing gold ions or gold complexes and then irradiated with an ultrasonic wave or an ionizing radiation or a method in which metal ions that provide a magnetic iron oxide are added to a solution containing gold ions or gold complexes and then irradiated with an ultrasonic wave or an ionizing radiation, due to the reason that the effects and the like of the present invention are more excellent. With the above irradiation, reduced gold nanoparticles are deposited on the surface of the magnetic iron oxide.

The solution containing gold ions can be obtained, for example, by dissolving a gold chloride salt in water, in hydrous alcohol or alcohol, such as methanol, ethanol, n-propanol, isopropanol, n-butanol, sec-butanol, or t-butanol, or in an acid such as hydrochloric acid, sulfuric acid, or nitric acid.

The concentration of the gold ion in the solution containing gold ions is not particularly limited; however, the preferred lower limit thereof is 1 µmol/L, and the preferred upper limit thereof is 1 mol/L. In a case the concentration thereof is less than 1 µmol/L, the desired composite particle is hardly obtained, and in a case where it exceeds 1 mol/L, the size of the gold particle becomes too large, or a large amount of particles made of only gold is generated. The more preferred lower limit thereof is 0.1 mmol/L, and the more preferred upper limit thereof is 10 mmol/L.

Examples of the solution containing the gold complex include an aqueous solution, hydrous alcohol, or alcohol solution of a compound in which a suitable ligand is coordinated with a gold ion.

The ligand is not particularly limited as long as it has an unshared electron pair or a negative charge. Specific examples thereof include monodentate ligands such as a halide ion (F⁻, Cl⁻, Br⁻, I⁻, or the like), a cyanide ion (CN⁻), ammonia (NH₃), and pyridine; didentate ligands such as ethylenediamine and an acetyl acetone ion; and hexadentate ligands such as an ethylenediamine tetraacetate ion.

As necessary, the solution containing the gold ion or gold complex may contain, for example, additives such as various water-miscible organic solvents, for example, a water-soluble polymer compound such as polyvinyl alcohol; a surfactant; alcohols; ethers such as tetrahydrofuran, diethyl ether, diisopropyl ether; polyols such as an alkylene glycol, a polyalkylene glycol, a monoalkyl ether or dialkyl ether thereof, and glycerin; and ketones such as acetone and methyl ethyl ketone.

Such additives are effective in accelerating the reduction reaction rate of the metal ion and adjusting the size of the metal particle to be generated.

The manufacturing method for the magnetic particle is not particularly limited, and, for example, the magnetic particle can be manufactured according to a method described in a physical vapor synthesis (PVS) method (see C. J. Parker, M. N. All, B. B. Lympany, US5514349A) or the like.

In addition, as the magnetic particle, for example, a commercially available product manufactured by Nanophase Technologies Corporation can be used.

In a case of manufacturing the composite particle according to the embodiment of the present invention, gold particles can be bound to the surface of the magnetic particles by being irradiated with an ultrasonic wave or an ionizing radiation.

The irradiation with the ultrasonic wave can usually be carried out under the conditions of a frequency of 10 kHz to 10 MHz and an output of 1 W or more. The ultrasonic irradiation is more preferably carried out in an inert gas replaced atmosphere, such as argon. Examples of the preferred irradiation conditions include conditions of a frequency of 200 kHz, an output of 200 W, and an irradiation time of 1 to 300 minutes.

The ionizing radiation includes a direct (primary) ionizing radiation and an indirect (secondary) ionizing radiation. The direct ionizing radiation is a charged particle beam such as an electron, a proton, or an α particle, and the indirect ionizing radiation is an uncharged particle beam such as a γ-ray (an electromagnetic wave), an X-ray, or a neutron beam. The wavelength of this ionizing radiation is preferably less than 1 nm, more preferably 0.1 nm or less, and particularly preferably 0.01 nm or less. In particular, as shorter the wavelength is, fine gold particles having the more uniform size tend to be generated in a short time.

The irradiation with the ionizing radiation can usually be carried out under the conditions of an absorption dose of 1 J/kg or more and preferably an absorption dose of 1 to 1,000,000 J/kg. In particular, in a case where a γ-ray is used as the ionizing radiation, the irradiation with the γ-ray is preferably carried out under conditions of a dose of 1 Gy or more. The preferred specific example of the γ-ray irradiation is an example in which for example, a cobalt 60 γ-ray source (γ-ray photon energy: 1.25 MeV) is used as the radiation source, and the irradiation is carried out under the conditions of a dose rate of about 3 kGy/h and an irradiation time of 1 to 18 hours.

The irradiation with the ionizing radiation is preferably carried out while stirring the solution in order to maintain the dispersed state of the magnetic particles. In the case of the ultrasonic irradiation, since the ultrasonic irradiation itself has a stirring effect, no particular stirring operation is required.

For the above-described manufacturing method, the ultrasonic irradiation is preferably used. With the ultrasonic irradiation, the support rate of gold particles on the core particle is increased, and a desired composite particle having good dispersibility can be obtained.

The temperature conditions at the time of the irradiation with the ionizing radiation or the ultrasonic wave are not particularly limited, and the irradiation is usually carried out at room temperature (normal temperature); however, cooling conditions and heating conditions of about 0°C to 100°C can be also adopted.

The dispersion liquid of the composite particle according to the embodiment of the present invention thus obtained can be used as it is as a dispersion liquid in the mixing step described later, or the composite particle according to the embodiment of the present invention dispersed in the dispersion liquid are separated and then dried. Then, it can be used as a powder in the mixing step described later.

Examples of the suitable manufacturing method for a composite particle according to the embodiment of the present invention are described in Mater. Res. Express 1 (2014) 035023, and "Synthesis of nanoparticle material by γ-ray in which gold and magnetic iron oxide are composited", Journal of the Japan Society of Powder and Powder Metallurgy, Vol. 51, No. 9, 680, and such a particle can be purchased, for example, from Act Nonpareil.

### [Mixing step]

The mixing step is a step of mixing a specimen that is capable of containing a test substance with a modified composite particle that is a composite particle modified with a first binding substance that is capable of binding to the test substance, to obtain a complex of the test substance in the specimen and the modified composite particle.

Here, the composite particle is a composite particle (the above-described composite particle according to the embodiment of the present invention) of a magnetic particle and a gold particle carried on a surface of the magnetic particle.

### <Specimen>

The specimen that is used in the mixing step is not particularly limited as long as it is a specimen that is capable of containing a test substance. Examples of such a specimen include a biological specimen, particularly a biological specimen of animal origin (particularly, of human origin) such as a body fluid (for example, blood, serum, plasma, spinal fluid, tear fluid, sweat, urine, pus, runny nose, or sputum) or excrement (for example, feces), an organ, a tissue, a mucous membrane or skin, a scraped test sample (a swab) that is conceived to contain these substances, a mouthwash, and an animal and a plant themselves or a dried substance thereof.

Examples of the test substance include a natural substance, a toxin, a hormone, a physiologically active substance such as a pesticide, an environmental pollutant, a virus (for example, an influenza virus), an antigen, and an antibody.

### (Pretreatment of specimen)

It is possible to use the above-described specimen as it is or in a form of an extraction liquid obtained by extracting the test specimen using an appropriate solvent for extraction, in a form of a diluent solution obtained by diluting an extraction liquid with an appropriate diluent, or in a form in which an extraction liquid has been concentrated by an appropriate method.

As the solvent for extraction, it is possible to use a solvent (for example, water, physiological saline, and a buffer solution) that is used in a general immunological analysis method, or a water-miscible organic solvent with which a direct antigen-antibody reaction can be carried out by being diluted with such a solvent.

### <Modified composite particle>

The modified composite particle is the above-described composite particle of the embodiment of the present invention, which is modified with the first binding substance that is capable of binding to the test substance. That is, the modified composite particle is the above-described composite particle of the embodiment of the present invention, and is a composite particle modified with the first binding substance that is capable of binding to the test substance.

In the modified composite particle, a portion (a site), which is to be modified with the first binding substance, of the above-described composite particle of the embodiment of the present invention is not particularly limited. For example, among the above-described composite particles of the embodiment of the present invention, the magnetic particle may be modified with the first binding substance, or the gold particle may be modified with the first binding substance. Among the above, it is preferable that the gold particle is modified with the above-described first binding substance due to the reason that the effects and the like of the present invention are more excellent.

### (First binding substance)

The first binding substance is not particularly limited as long as it is a compound having an affinity for a test substance, and examples thereof include an antibody that specifically binds to a test substance consisting of an antigen; an antigen that specifically binds to a test substance consisting of an antibody; and an aptamer that binds to a test substance composed of a protein, a low molecular weight compound, and the like.

In the chromatography of the embodiment of the present invention, it is preferable that the test substance is an antigen and the first binding substance is an antibody.

The above-described antibody is not particularly limited. However, for example, it is possible to use antisera prepared from animal sera immunized with the test substance, an immunoglobulin fraction purified from antisera, a monoclonal antibody obtained by cell fusion using animal spleen cells immunized with the test substance, or a fragment thereof [for example, F(ab')2, Fab, Fab', or Fv]. Preparation of these antibodies can be carried out by a conventional method.

### (Method for manufacturing modified composite particle)

The method for manufacturing the modified composite particle is not particularly limited, and a known method in the related art can be used. Examples thereof include a physical binding method such as a method in which the above-described composite particles according to the embodiment of the present invention are immersed in a buffer solution containing an antibody and incubated at a predetermined temperature for a predetermined period of time or a chemical bonding method such as a method in which an SH group is introduced into an antibody, and the fact that gold and an SH group are chemically bonded is utilized so that the SH bond of the antibody is cleaved to generate an Au-S bond on the Au surface when the antibody approaches the above-described composite particle according to the embodiment of the present invention, whereby the antibody is immobilized. Among them, chemical bonding is preferable due to the reason that the effects and the like of the present invention are more excellent.

### <Mixing of specimen and modified composite particle>

In the mixing step, the specimen and the modified composite particle are mixed.

Accordingly, in a case where the specimen contains a test substance, the test substance in the specimen reacts with the first binding substance of the modified composite particle, and thereby a complex of the test substance and the modified composite particle is formed in the specimen. On the other hand, in a case where the specimen does not contain a test substance, the complex is not formed.

### [Developing step]

The developing step is a step of developing the complex obtained in the mixing step on an insoluble carrier having a reaction site at which a second binding substance capable of binding to the test substance is immobilized.

### <Insoluble carrier>

The above-described insoluble carrier is an insoluble carrier having a reaction site (a test line) at which a second binding substance that is capable of binding to the test substance is immobilized. The insoluble carrier may have a plurality of test lines depending on the kinds of the test substance. In addition, the insoluble carrier may have a control line on the downstream side of the test line in order to confirm the developing of the complex.

### (Insoluble carrier)

The insoluble carrier is preferably a porous carrier. A nitrocellulose membrane, a cellulose membrane, an acetyl cellulose membrane, a polysulfone membrane, a polyether sulfone membrane, a nylon membrane, a glass fiber, a non-woven fabric, a cloth, a thread, and the like are particularly preferable.

### (Second binding substance)

Specific examples and a suitable aspect of the second binding substance are the same as those of the above-described first binding substance. The second binding substance may be the same as or different from the above-described first binding substance; however, in a case where the first binding substance is an antibody, a paratope that recognizes a test substance or an epitope of the test substance (the antigen) recognized by the antibody which is the second binding substance is preferably different from that of the antibody which is the first binding substance.

### <Developing of complex>

In the developing step, the complex is preferably developed on the insoluble carrier. For example, the specimen containing the complex obtained in the mixing step is allowed to flow from one end of the insoluble carrier to develop the specimen containing the complex in the horizontal direction of the insoluble carrier.

As described above, in a case where the composite particles of the embodiment of the present invention (unreacted composite particles) which did not react with the test substance are present in the specimen obtained after the mixing step, the unreacted composite particles are also developed together with the complex. In addition, in a case where the specimen does not contain a test substance, a complex is not formed in the mixing step, and the unreacted composite particles are developed.

### [Capturing step]

The capturing step is a step of capturing the complex at the reaction site of the insoluble carrier.

As described above, since the second binding substance that is capable of binding to a test substance is immobilized at the reaction site of the insoluble carrier, the complex (the complex of a test substance and a modified composite particle) developed on the insoluble carrier in the developing step is captured at the reaction site (the test line) of the insoluble carrier.

Due to having magnetic particles, the captured complex is magnetically detected. In addition, the captured complex is visible since it is colored by surface plasmon of a gold particle and the like. In addition, it is also possible to estimate the concentration of the captured complex using an image analysis device or the like. In this manner, the test substance in the specimen can be detected.

In a case where a specimen does not contain a test substance, the complex is not formed, and thus the complex is not captured at the reaction site of the insoluble carrier and is not detected.

### [Silver amplification step]

The silver amplification step is a step of silver-amplifying the complex captured in the capturing step.

The silver amplification step is a step of forming large silver particles in the complex captured at the reaction site of the insoluble carrier by providing silver ions to the insoluble carrier after the capturing step. More specifically, it is a step in which silver ions are reduced using gold particles of the complex as a catalyst to form silver particles (for example, a diameter of 10 µm or more).

This significantly improves the magnetic detection sensitivity of the captured complex. In addition, the visibility and the signal to noise (SN) ratio are significantly improved. That is, the optical detection sensitivity is also significantly improved.

### <Suitable aspect>

The method for providing silver ions to the insoluble carrier after the capturing step is not particularly limited; however, it is preferably a method in which the following reducing agent solution and the following silver amplification solution are used, due to the reason that the effects and the like of the present invention are more excellent.

Further, in addition to the reducing agent solution and the silver amplification solution, a washing solution may be used to wash the complex remaining on the insoluble carrier except for the specific binding reaction. The reducing agent solution may also serve as a washing solution.

### (Reducing agent solution)

The reducing agent solution contains a reducing agent capable of reducing silver ions. As the reducing agent capable of reducing silver ions, any inorganic or organic material or a mixture thereof can be used as long as it can reduce silver ions to silver. Preferred examples of inorganic reducing agents include reducing metal salts and reducing metal complex salts which are capable of changing an atomic value with metal ions such as Fe²⁺, V²⁺, or Ti³⁺. In a case in which an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), and therefore detoxification is possible. In the present invention, it is preferable to use such an inorganic reducing agent, and as a more preferable aspect of the present invention, it is preferable to use a metal salt of Fe²⁺ as the reducing agent.

It is also possible to use a main developing agent used in a light-sensitive silver halide photographic material of a wet-type (such as methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), and leuco colorants), and other materials obvious to those who are skilled in the technology in the present field, such as a material disclosed in US6020117A.

As the reducing agent, an ascorbic acid reducing agent is also preferable. The useful ascorbic acid reducing agent includes ascorbic acid, an analog thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-e0rythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type, and the like. Particularly preferred examples thereof include D-, L-, or D,L-ascorbic acid (and an alkali metal salt thereof) or isoascorbic acid (or an alkali metal salt thereof), and a sodium salt is a preferred salt. A mixture of these reducing agents can be used as necessary.

Due to the reason that the effects and the like of the present invention are more excellent, the reducing agent solution is preferably allowed to flow so that the angle between the developing direction in the developing step and the developing direction of the reducing agent solution is 0 degrees to 150 degrees, and more preferably allowed to flow so that the angle between the developing direction in the developing step and the developing direction of the reducing agent solution is 0 degrees to 135 degrees.

Examples of the method for regulating the angle between the developing direction in the developing step and the developing direction of the reducing agent solution include the method described in Examples of JP2009-150869A.

### (Silver amplification solution)

The silver amplification solution is a solution containing a compound containing silver ions. As the compound containing silver ions, it is possible to use, for example, organic silver salts, inorganic silver salts, or silver complexes. The compound is preferably a silver ion-containing compound having a high solubility in solvents such as water, and examples thereof include silver nitrate, silver acetate, silver lactate, silver butyrate, silver thiosulfate, and the like. Silver nitrate is particularly preferable. Silver complexes are preferably silver complexes coordinated with ligands having a water-soluble group such as a hydroxyl group or a sulfone group, and examples thereof include silver hydroxythioether and the like.

As the silver, the organic silver salt, the inorganic silver salt, or the silver complex is preferably contained at a concentration of 0.001 mol/L to 5 mol/L, preferably 0.005 mol/L to 3 mol/L, and more preferably 0.01 mol/L to 1 mol/L, in the silver amplification solution.

Examples of the auxiliary agent of the silver amplification solution include a buffer, a preservative such as an antioxidant or an organic stabilizer, and a rate regulating agent. As the buffer, it is possible to use, for example, a buffer formed of acetic acid, citric acid, sodium hydroxide, or salts of any of these substances, or tris(hydroxymethyl)aminomethane or other buffers used in general chemical experiments. These buffers are appropriately used to adjust the pH of the amplification liquid to an optimum pH thereof. In addition, as the antifogging agent, an alkyl amine can be used as an auxiliary agent, and dodecyl amine is particularly preferable. In addition, a surfactant can be used for the intended purpose of improving the solubility of this auxiliary agent, and C₉H₁₉-C₆H₄-O-(CH₂CH₂O)₅₀H is particularly preferable.

Due to the reason that the effects and the like of the present invention are more excellent, the silver amplification solution is preferably allowed to flow from the direction opposite to that of the developing step described above and more preferably allowed to flow so that the angle between the developing direction in the developing step and the developing direction of the reducing agent solution is 45 degrees to 180 degrees.

Examples of the method for regulating the angle between the developing direction in the developing step and the developing direction of the silver amplification solution include the method described in Examples of JP2009-150869A.

### [Collection step]

The immunochromatography according to the embodiment of the present invention may further include a collection step of collecting the modified composite particle or complex using magnetism, before the developing step described above.

This makes it possible to carry out operations such as increasing the concentration of the modified composite particle or the complex in the specimen, separating the modified composite particle or the complex, and the like. In addition, this makes it possible to significantly improve the detection sensitivity and the signal to noise (SN) ratio.

The method for collecting a modified composite particle or a complex by using magnetism is not particularly limited, and examples thereof include a method for putting the specimen obtained after the mixing step into a microtube installed on a magnetic stand. Examples

Hereinafter, the present invention will be described in more detail with reference to examples, but the present invention is not limited thereto.

### [Preparation of sample solution]

A specimen (a sample solution) capable of containing a test substance (the influenza virus) was prepared using a Quick S-Influ A·B "SEIKEN" negative/positive control solution (manufactured by DENKA SEIKEN Co., Ltd.).

Specifically, the above positive control solution was diluted at dilution rates of 1/10, 1/20, 1/40, 1/80, 1/160, 1/320, 1/640, and 1/1,280 (mass ratio) with a phosphate buffered salt (PBS) buffer containing 1% by mass bovine serum albumin (BSA), and the resultant solutions were used as specimens (sample solutions) that were capable of containing the test substance (the influenza virus).

### [Example 1]

Immunochromatography of Example 1 was carried out as follows.

### <Mixing step>

A gold magnetic nanoparticle "GoldMAN" manufactured by Act Nonpareil (a composite particle of an iron oxide particle (average particle diameter: about 40 nm) and a gold particle (average particle diameter: about several nanometers) carried on the surface of the iron oxide particle, number of carried gold particles: 30, average particle diameter: about 50 nm) (corresponding to the above-described composite particle according to the embodiment of the present invention) was purified and buffer-exchanged, and then mixed with an anti-influenza A type antibody (corresponding to the above-described first binding substance) to prepare gold magnetic nanoparticle (corresponding to the above-described modified composite particle) (hereinafter, also referred to as the "modified composite particle 1") modified with the anti-influenza A type antibody. At that time, the modified composite particle 1 was collected using magnetism, and the excessive antibody was eliminated.

Next, each of the sample solutions prepared as described above was mixed with the modified composite particle 1 prepared as described above to obtain a complex of the test substance (the influenza virus) in each of the sample solutions and the modified composite particle 1.

The mixing step may be carried out, for example, by preparing a composite particle holding pad that is obtained by coating one glass fiber pad (Glass Fiber Conjugate Pad, manufactured by Millipore Corporation) cut into 8 mm × 150 mm with a composite particle that is modified with the first binding substance capable of binding to a test substance, immobilizing on a membrane, and adding a specimen capable of containing a test substance to the pad.

### <Developing step>

A nitrocellulose membrane (corresponding to the insoluble carrier described above) having a test line on which an anti-influenza A monoclonal antibody for immobilization was immobilized was prepared.

Next, the specimen containing the complex obtained in the mixing step was allowed to flow from one end of the nitrocellulose membrane to develope the specimen in the horizontal direction of the nitrocellulose membrane.

In Fig. 1, a schematic view (a plan view) of the prepared nitrocellulose membrane 10 is illustrated. Here, 1 is the position where a specimen containing a complex is dropped, A is the developing direction in the developing step, and a is the test line.

### <Capturing step>

The complex in the specimen that is developed in the developing step is captured on the test line.

### <Silver amplification step>

The silver amplification step was carried out as follows.

### (Preparation of reducing agent solution)

23.6 mL of an aqueous solution of 1 mol/L iron nitrate, which was produced by dissolving iron (III) nitrate nonahydrate (manufactured by FUJIFILM Wako Pure Chemical Corporation) in water, and 13.1 g of citric acid (manufactured by FUJIFILM Wako Pure Chemical Corporation) were dissolved in 290 g of water. After all of the substances were dissolved, 36 ml of nitric acid (10% by mass) was added thereto while stirring with a stirrer, 60.8 g of ammonium iron (II) sulfate hexahydrate (manufactured by FUJIFILM Wako Pure Chemical Corporation) was added thereto, and the resultant solution was used as the reducing agent solution.

### (Preparation of silver amplification solution)

8 mL of a silver nitrate solution (including 10 g of silver nitrate) and 24 mL of an aqueous solution of 1 mol/L iron nitrate were added to 66 g of water. Further, this solution was mixed with a solution obtained by dissolving 5.9 mL of nitric acid (10% by mass), 0.1 g of dodecyl amine (manufactured by FUJIFILM Wako Pure Chemical Corporation), and 0.1 g of a surfactant C₁₂H₂₅-C₆H₄-O-(CH₂CH₂O)₅₀H in 47.6 g of water in advance, and the resultant solution was used as the silver amplification solution.

### (Developing of reducing agent solution)

In the nitrocellulose membrane after the capturing step, the reducing agent solution prepared as described above was allowed to flow from the same direction as that of the developing step described above. At that time, the angle between the developing direction A in the developing step and the developing direction of the reducing agent solution was set to be 0 degrees.

### (Developing of silver amplification solution)

Then, the silver amplification solution prepared as described above was allowed to flow from the direction opposite to the developing direction in the developing step. At that time, the angle between the developing direction A in the developing step and the developing direction of the reducing agent solution was set to be 180 degrees.

### <Evaluation>

The optical detection sensitivity and the magnetic detection sensitivity were evaluated as follows.

### (Optical detection sensitivity)

ΔOD (the optical density) on the test line of the nitrocellulose membrane after the silver amplification step was measured using LAS2000 (manufactured by FUJIFILM Corporation).

Then, the dilution rate of a sample solution of which ΔOD was smallest among the sample solutions of which ΔOD could be measured was determined. The results are shown in Table 1. It means that the smaller the value (the dilution rate), the more detectable the test substance even in the case of the specimen having a low concentration of the test substance (the influenza virus), which means that the optical detection sensitivity is excellent.

### (Magnetic detection sensitivity)

The magnetic detection sensitivity of the nitrocellulose membrane after the silver amplification step was evaluated using a GMR sensor (a differential magnetic field sensor, manufactured by NVE Corporation). Specifically, regarding the case where a sample solution having a dilution rate of 1/20 was used as the sample solution, the measurement was carried out to obtain the magnetism quantity a [mV] in a case where the sensor was installed on the test line a, the magnetism quantity b [mV] in a case where the sensor was installed at the position b close to the dropping position in the developing step by about 5 mm from the test line, and the magnetism quantity c [mV] in a case where the sensor was installed at the position c far from the dropping position in the developing step by about 5 mm from the test line, and then (a - b)/a and (a - c)/a were calculated. The results are shown in Table 1. It means that the larger (a - b)/a and (a - c)/a, the more excellent the magnetic detection sensitivity. That these numerical values increase means that no magnetic particles are present in other places except the position directly on the test line. In general, particles present other places except for the test line increase the concern about false positiveness. As a result, it means that in the detection method in which the above numerical values are large, false positiveness is small, that is, sensitivity is high.

### [Comparative Example 1]

Immunochromatography was carried out according to the same procedure and evaluated in the same manner as in Example 1 except that in the mixing step, a gold nanoparticle (average particle diameter: about 50 nm) manufactured by Sigma-Aldrich Co., LLC was used instead of the gold magnetic nanoparticle "GoldMAN" manufactured by Act Nonpareil and excess antibody was eliminated by centrifugation. It is noted that magnetic detection was not possible (due to the low detection limit).

### [Comparative Example 2]

Immunochromatography was carried out according to the same procedure as in Example 1 except that the silver amplification step was not carried out, and the evaluation was carried out in the same manner. It is noted that optical detection was not possible (due to the low detection limit).

### [Comparative Example 3]

Immunochromatography was carried out according to the same procedure and evaluated in the same manner as in Example 1 except that in the mixing step, a gold nanoparticle (average particle diameter: about 50 nm) manufactured by Sigma-Aldrich Co., LLC was used instead of the gold magnetic nanoparticle "GoldMAN" manufactured by Act Nonpareil, an excess antibody was eliminated by centrifugation, and the silver amplification step was not carried out. It is noted that magnetic detection was not possible (due to the low detection limit).

It is noted that the number of composite bodies in the specimen allowed to flow in the developing step was set to be about the same between sample solution of Examples and Comparative Examples. For example, in Example 1 and Comparative Examples 1 to 3 in which a sample solution having a dilution rate of 1/10 was used, the number of composite bodies in the specimen allowed to flow in the developing step was set to be about the same.

**[Table 1]**

| | | Example 1 | Comparative Example 1 | Comparative Example 2 | Comparative Example 3 |
|---|---|---|---|---|---|
| Label | | Composite particle | Gold nanoparticle | Composite particle | Gold nanoparticle |
| Silver amplification step | | Present | Present | Absent | Absent |
| Optical detection sensitivity | | 1/1,280 | 1/640 | - | 1/20 |
| Magnetic detection sensitivity | (a - c)/a | 0.91 | - | 0.35 | - |
| | (a - b)/a | 0.95 | - | 0.39 | - |

| | | | | | |
|---|---|---|---|---|---|
| ("-" means that detection is not possible) | | | | | |

As can be seen from Table 1, the chromatography of Example 1, which uses the composite particle according to the embodiment of the present invention as a label and includes the silver amplification step, exhibits extremely excellent magnetic detection sensitivity, as compared with the chromatography (corresponding to JP2007-205911A) of Comparative Example 2, which uses the composite particle according to the embodiment of the present invention as a label but does not include the silver amplification step.

Further, the chromatography of Example 1, which uses the composite particle according to the embodiment of the present invention as a label and includes the silver amplification step, exhibits excellent optical detection sensitivity, as compared with that of Comparative Example 1, which includes the silver amplification step but does not use the composite particle (uses the gold nanoparticle) according to the embodiment of the present invention as a label.

In addition, when the same chromatography was also carried out using a particle modified with an anti-influenza B type antibody instead of the particle modified with the anti-influenza A type antibody and using a mixture (1: 1 by mass ratio) of the particle modified with the anti-influenza A type antibody and the particle modified with the anti-influenza B type antibody instead of the particle modified with the anti-influenza A type antibody and evaluated in the same manner, the same tendency as in Table 1 was confirmed.

### Explanation of References

- 1: a: position where a specimen containing a complex is dropped
- 10:: nitrocellulose membrane

## Claims

1. Immunochromatography comprising:
a mixing step of mixing a specimen that is capable of containing a test substance with a modified composite particle that is a composite particle modified with a first binding substance that is capable of binding to the test substance, to obtain a complex of the test substance in the specimen and the modified composite particle;
a developing step of developing the complex on an insoluble carrier having a reaction site at which a second binding substance capable of binding to the test substance is immobilized;
a capturing step of capturing the complex at the reaction site of the insoluble carrier; and
a silver amplification step of silver-amplifying the complex captured in the capturing step,
wherein the composite particle is a composite particle of a magnetic particle and a gold particle carried on a surface of the magnetic particle.

2. The immunochromatography according to claim 1,
wherein a material of the magnetic particle is iron oxide.

3. The immunochromatography according to claim 1 or 2, further comprising a collection step of collecting the modified composite particle or the complex using magnetism, before the developing step.

4. The immunochromatography according to any one of claims 1 to 3,
wherein the number of carried gold particles of the composite particle is 1 or more and 300 or less.

5. The immunochromatography according to any one of claims 1 to 4,
wherein in the composite particle, an average particle diameter of the gold particles is smaller than an average particle diameter of the magnetic particles.

6. The immunochromatography according to any one of claims 1 to 5,
wherein an average particle diameter of the composite particles is 10 nm to 300 nm.

7. The immunochromatography according to any one of claims 1 to 6,
wherein the silver amplification step is a step of silver-amplifying the complex captured in the capturing step, using a reducing agent solution and a silver amplification solution.

8. The immunochromatography according to claim 7,
wherein an angle between a developing direction of the reducing agent solution in the silver amplification step and a developing direction of the complex in the developing step is 0 degrees to 150 degrees.

9. The immunochromatography according to claim 7 or 8,
wherein an angle between a developing direction of the silver amplification solution in the silver amplification step and a developing direction of the complex in the developing step is 45 degrees to 180 degrees.

10. The immunochromatography according to any one of claims 1 to 9,
wherein both optical detection and magnetic detection are possible.
